# EUROPEAN PATENT SPECIFICATION

(11) **EP 3 426 022 B1**
(45) Date of publication and mention of the grant of the patent: **07.09.2022**
(21) Application number: 17762608.2
(22) Date of filing: 27.02.2017
(51) Int. Cl.: A01K 11/00, A61B 10/02, A61B 10/00

(54) **TISSUE SAMPLING CARTRIDGE AND METHOD**
GEWEBEPROBENKASSETTE UND VERFAHREN
CARTOUCHE D'ÉCHANTILLONNAGE DE TISSU ET PROCÉDÉ

(30) Priority: 08.03.2016 US 201662305498 P; 31.03.2016 NZ 16718587; 31.03.2016 NZ 16718588; 31.03.2016 US 201662316499 P; 29.04.2016 NZ 16719578; 02.11.2016 NZ 16725834; 02.11.2016 NZ 16725835; 02.11.2016 NZ 16725836; 02.11.2016 NZ 16725837
(43) Date of publication of application: 16.01.2019
(73) Proprietor: Snpshot Trustee Limited, Auckland 1010 (NZ)
(72) Inventor: BLADEN, Rory, Chicago, IL (US); BLADEN, Roy Victor, Auckland 0793 (NZ); GARDNER, Michael Stuart, Auckland 1050 (NZ)
(74) Representative: Algemeen Octrooi- en Merkenbureau B.V.
(86) International application number: PCT/IB2017/051120
(87) International publication number: WO 2017/153866

(56) References cited:
- EP-B1- 1 060 662
- WO-A1-2014/196876
- WO-A1-2015/056225
- WO-A1-2015/056229
- WO-A1-2016/107825
- US-A1- 2007 021 684
- US-A1- 2010 094 170
- US-A1- 2010 210 011
- US-A1- 2013 211 287

## Description

### FIELD OF THE INVENTION

The present invention relates to tissue sampling, and in particular though not solely to a cartridge carrying components to facilitate the removal of a sample from an animal.

### BACKGROUND TO THE INVENTION

Tissue sampling for the purposes of recording an animal's DNA and for subsequent tracking purposes is becoming more prevalent. For example, farmers may remove a tissue sample from the ear of an animal on the farm. The removed sample may then be in some way associated with the animal by way of a unique identifier, and may then be shipped to a laboratory for analysis. However, difficulties are still arising in respect of sample security and the prevention of tampering of the sample on the farm or during transit to the laboratory, as well as issues around cross-contamination of the sample between animals.

US 2007/021684 discloses a tissue sampler with integral sample collector and able to retain a removable sample container. The tissue sampler having a flexible bridge allowing the sample collector to move through a sample, to be finally be located in the sample container whilst the sample collector is connected to said bridge.

WO 2015/056229 discloses a tissue sampler comprising a removable magazine. A removable sample collector is loaded into the removable magazine. The sample collector is driven from the removable magazine into a sample container that is held by the tissue sampler.

It is therefore an object of the present invention to provide a sampling cartridge and/or sampler with which the cartridge is removably associated that overcomes the abovementioned disadvantages, and/or at least provides the public with a useful choice.

Where used in this specification tissue means any part of a living thing, particularly any part made up of similar cells, or any part or parts that perform a similar function. Tissue preferably refers to any form of biological sample, from plants and animals particularly, including pigs, goats, cattle, sheep, poultry, and fish. Biological samples may include for example, animal tissue such as flesh, blood, hair, fur, saliva, sweat, urine, etc, or plant tissue such as leaves, bark, roots or wood, or any other part of a plant or animal but particularly those that are made up of similar cells, or which perform a similar function.

The cartridge of the present invention may be used in tissue sampling at least for either or both of production animals and companion animals. It is anticipated that production animals may include but not be limited to bovine, pigs, deer and sheep. Further it is anticipated that companion animals may include but not be limited to horses, cats and dogs.

Accordingly in a first aspect the present invention consists in a sampling cartridge according to claim 1.

Preferably the cartridge is adapted and configured to be loadable and unloadable to and from a sampler device that includes a driver actuable to cause said in alignment sample collector to move from sample collector holding region across the gap and into the sample storage container.

Preferably the sampling cartridge body is adapted and configured to shield the sampler device at the gap from contact with said item to be sampled when placed at the gap.

Preferably the sampling cartridge body is adapted and configured to shield the sampler device at the gap from contact with said item to be sampled when placed at the gap, and any exudates expressed or expelled from said item during sampling.

Preferably the cartridge comprising a bridge section extending between said storage container holding region and said sample collector holding region, said bridge section spanning said gap.

Preferably said bridge section provides said shield function.

Preferably said bridge section is the only connection of the cartridge between said storage container holding region and said sample collector holding region.

Preferably said bridge section defines a trough to said gap.

Preferably the gap is substantially U shaped, including two facing side walls and a base defined by said bridge section and said storage container holding region and said sample collector holding region.

Preferably the gap is substantially U shaped.

Preferably said storage container holding region and said sample collector holding region are contiguous respective opposed sides of said gap.

Preferably said storage container holding region comprises a cavity of said sampling cartridge body in which at least part of said storage container is located.

Preferably said cavity is a bore.

Preferably a passage leads from said cavity to said gap and via which sample collector can move to engage with said storage container.

Preferably the passage is blocked pre-sampling.

Preferably the passage is blocked by a removable seal.

Preferably the removable seal is a film, preferably able to be ruptured and/or peeled away from the passage to expose the passage to said gap.

Preferably the passage includes a mouth opening presented at said gap at where said sample collector enters the passage, upon the taking of a sample from an item to be sampled placed at the gap, to advance for engagement via on opening of and into said storage container.

Preferably the material about said mouth is harder than said material of the storage container about its said opening.

Preferably at said mouth opening said sampling cartridge body together with said sample collector act as a punch and die set, the mouth opening acting as the die acting in shear with the sample collector acting as said punch to facility the removal of a sample from the item to be sampled.

Preferably said mouth opening is of a shape and configuration to allow said sample collector to enter into said passage in a snug fit manner.

Preferably passage extends between its said opening and said cavity at or adjacent where an opening of said storage container is presented.

Preferably said cavity is closed by a removable closure that together with said cavity encloses said storage container in said sampling cartridge body.

Preferably said removable closure is able to be removed to open said cavity and expose said storage container to allow said storage container to be removed from said sampling cartridge body.

Preferably said removable closure is engaged to said sampling cartridge body in a tamper evident manner.

Preferably said removable closure is threadingly engaged to said sampling cartridge body.

Preferably said sampling cartridge body includes a second cavity in which said sample collector is at least partially located, pre-sampling.

Preferably said sample collector is entirely located in said second cavity.

Preferably said second cavity includes an opening to said gap via which said sample collector can pass from said cavity towards said storage container during sampling.

Preferably said opening of said second cavity is sealed by a removable seal (e.g. a film seal of cap or plug).

Preferably said second cavity is fully sealed pre sampling to enclose said sample collector therein.

Preferably the sampling cartridge body carries only one sample collector and one sample storage container.

Preferably at least one of the components selected from the following list:
a. Removable closure,
b.Storage container (preferably the storage tube),
c. Sample collector (preferably the plunger)
d.Sampling cartridge body,
comprises at least one form of visible and/or machine readable identification.

Preferably the identification is a unique identification.

Preferably the identification is not the same on each of said components but are preferably matched.

Preferably the identification is provided in the form selected from one of more of an electronic identification (EID) or visible identification such as a bar code, number, QR code, alphanumeric string or the like.

Preferably the sample collector comprises a punch presenting at one end a cutter to cut and hold a sample from the item as it passes there through, the punch having a bore at where a sample, once cut is able to locate with said punch.

Preferably said sample collector further comprises a plunger slideably supported at said bore to be able to move between a retracted position and an advanced position relative said punch, the movement from the retracted position to the advanced position causing, in use, the cutter held sample to be displaced from the cutter.

Preferably the sample collector is generally of circular cross section and elongate and straight, the cutter located at one end of the collector.

Preferably the punch is generally of circular cross section and elongate and straight, the cutter located at one end of the collector.

Preferably the plunger protrudes from the end of the punch opposite the cutter.

Preferably the plunger is rod shaped.

Preferably the punch is to seal the opening (or passage extending therefrom) of a sample storage container with which the sample collector is to engage after the sample has been taken.

Preferably the storage container includes a mouth opening leading to a storage region of the storage container, the mouth opening able to receive the sample collector after the sample collector has been driven through the item and is carrying a sample.

Preferably the storage container is adapted and configured to retain the sample collector (preferably the punch) once received at said mouth opening in a manner to snugly hold the sample collector to said storage container (preferably sealing the mouth opening).

Preferably said storage container (preferably at or adjacent said mouth opening) includes a surface or surfaces that allow the sample collector to seal the containment region of the storage container.

Preferably said storage container comprises a container body defining a containment region and a cap removably engaged to the container body, a mouth opening to said container body provided at a passage defined by said cap.

Preferably said passage and said sample collectors are adapted and configured to engage in a snap-fit manner to retain the sample collectors with the cap and seal the containment region.

In a further aspect the present invention consists of a method of taking and storing a sample from an item using the sampling cartridge according to claim 13.

Preferably the identification is matched to identification of said sampling cartridge.

Preferably said sampling cartridge is removed from the sampling device after the sample has been removed from the item to be taken, the sampling cartridge carrying the sample collector engaged with the storage container at one side of the gap.

Preferably the removed sampling cartridge is then sent to a laboratory for sample analysis.

Preferably the identification and the item and the identification of the sampling cartridge are reviewed after sampling to determine these correspond to the matched identification.

In a further aspect the present invention may be said to be a kit of a sampler and sampling cartridge as claimed wherein the sampler comprises an actuable driver and a receptacle configured to releaseably receive said cartridge to said sampler said driver presented and actuable to cause said in alignment sample collector to move from sample collector holding region across the gap and into the sample storage container.

Preferably the sampler comprises a U shape receptacle at where the cartridge is receivable.

Preferably the sampling cartridge body is adapted and configured to shield the sampler at the gap from contact with said item to be sampled when placed at the gap, and any exudates expressed or expelled from said item during sampling.

In a further aspect the present invention may be said to be a sampler for use with the cartridge according to claim 1.

Preferably the sampling cartridge body is disposable (and is preferably disposed of after the same retaining storage container has been removed from it).

Preferably the sampling cartridge engages with said sampling device in a releasable snap fit or other locked manner.

In yet a further aspect the present invention may broadly be said to be, as a set, a sampling device and a plurality of sampling cartridges according to claim 1, each individually and successively loadable with said sampling device for the purposes of each collecting one sample from one of more items to be sampled.

Preferably one (or more) of the invention(s) herein described may be used for production animals and for companion animals.

Preferably one (or more) of the invention(s) herein described may only be used for production animals.

Preferably one (or more) of the invention(s) herein described may only be used for companion animals.

Preferably production animals include but are not limited to bovine, pigs, deer and sheep.

Preferably companion animals include but are not limited to horses, cats and dogs.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner.

This invention may also be said broadly to consist in the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

The invention consists in the foregoing and also envisages constructions of which the following gives examples only. The invention is defined by the claims.

### BRIEF DESCRIPTION OF THE DRAWINGS

A preferred form of the invention will now be described with reference to the accompanying drawings in which:
Figure 1 is a perspective view of a sampler device and sampling cartridge associated therewith,
Figure 2 is an alternative perspective view of a sampler device and sampling cartridge associated therewith,
Figure 3 is a view of the sampling device and sampling cartridge in a pre- or post-engaged condition showing the removable nature of the sampling cartridge,
Figure 4 is a perspective view of Figure 3,

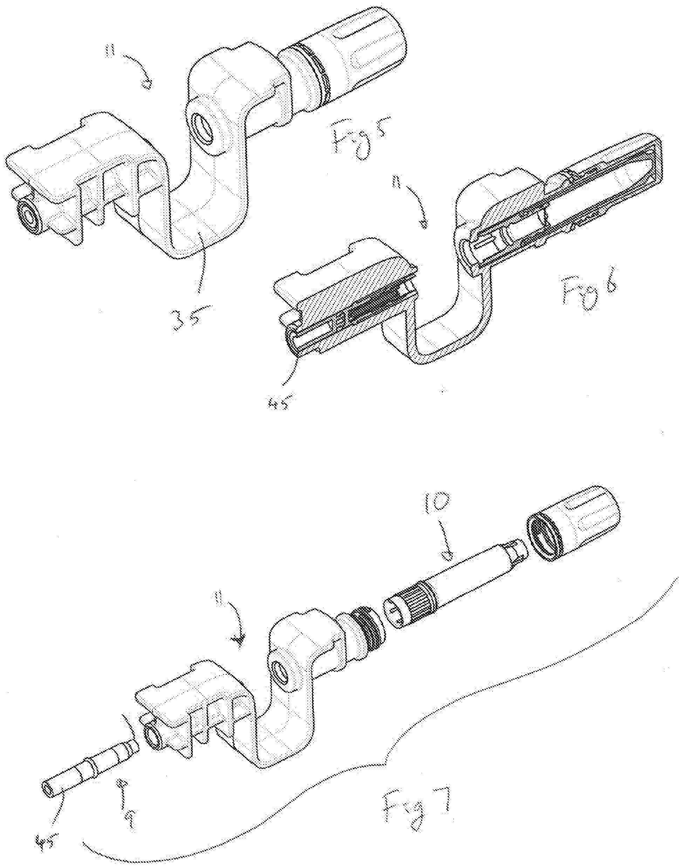

Figure 5 is a perspective view of the sampling cartridge,
Figure 6 is a perspective and partial sectional view of the sampling cartridge,
Figure 7 is an exploded view of the sampling cartridge,
Figure 8 is a sectional view of the sampling cartridge in a preloaded and ready for use condition,
Figure 9 is a sectional view of the sampling cartridge in a condition where the sample collector has advanced from its stored condition towards its sample storage container registered condition, and immediately prior to penetrating an item to be sampled,
Figure 10 is a sectional view of the sampling cartridge where the sample collector has moved from its storage condition and to its sample storage container registered condition,
Figure 11 illustrates the sampling cartridge engaged with the sampling device and parts of the internal mechanism of the sampling device wherein the sampling cartridge is in its pre-use condition,
Figure 12 shows the mechanism of the sampling device with its driver having driven the sample collector towards and into the storage container.

### DETAILED DESCRIPTION OF THE INVENTION

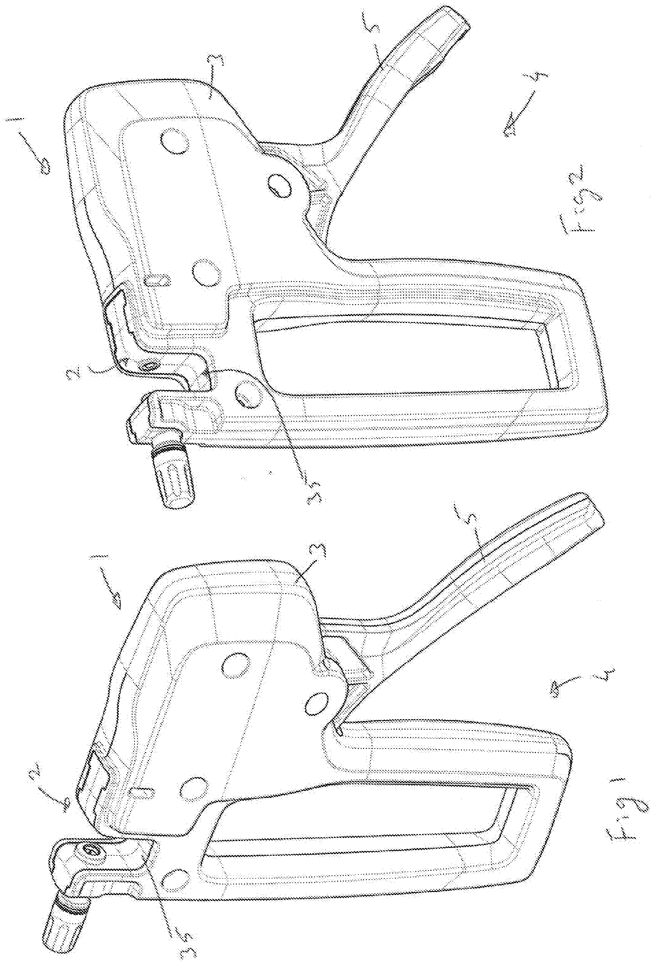

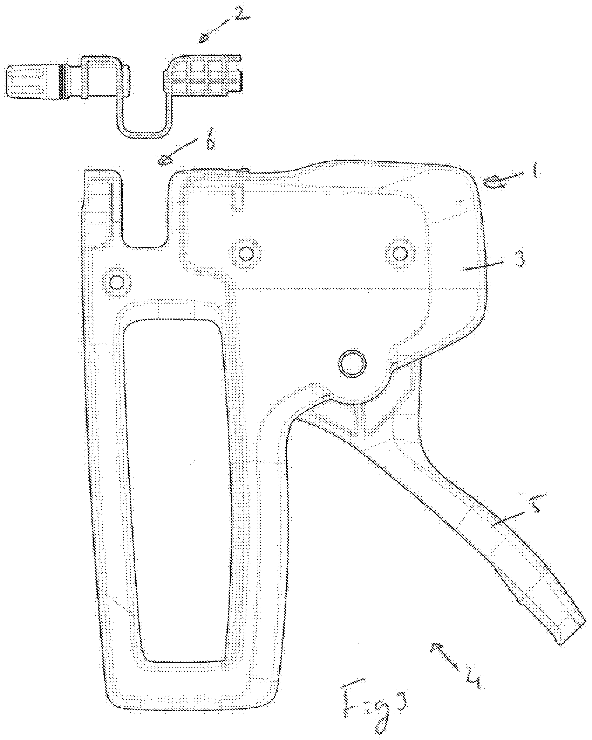

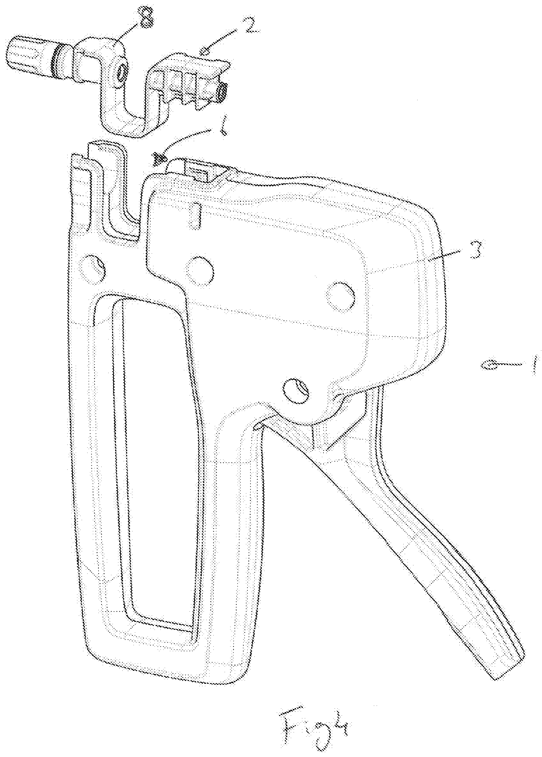

With reference to Figure 1, there is shown a sampling device (1) and a sampling cartridge (2). The sampling device includes a body (3) that includes a handle region (4) including a trigger (5). The trigger can be manipulated by a user to cause a sample to be taken and which will hereinafter be described in more detail. The sampling device (1) includes a sampling cartridge receptacle region (6) as can be seen in Figures 3 and 4.

The receptacle region is able to receive the sampling cartridge in a removable manner. Once received at the receptacle region, the cartridge is securely held by the sampling device. The sampling device can receive multiple cartridges in succession. The receptacle is adapted and configured together with the sampling cartridge (2), to allow the sampling cartridge to register with the body (3) of the sampling device (1) in a manner to present the sampling components of the sampling cartridge in an appropriate location relative to the driver (7) of the sampling device (1). In the preferred form, the receptacle region (6) and the sampling cartridge (2) are shaped and configured so that a snap-fit or close tolerance or interference fit relationship can be established between the body (3) and the sampling cartridge (2) when the sampling cartridge is full registered at the receptacle region (6) of the body (3). Such a relationship will ensure that the sampling cartridge is sufficiently securely engaged to the body (3) yet removeable therefrom upon a pulling or prying force being applied such as by a user's hand or fingers. This may be important because once loaded with the sampling device (1), the cartridge should remain engaged therewith until a sample has been taken whereafter the sampling cartridge can then be removed by the user from the body (3). The sampling cartridge loaded sampling device (1) may be dropped or placed on a surface or hung from a peg or otherwise manoeuvred, prior to a sampling being taken, and it is therefore important for the sampling cartridge to remain securely engaged with the sampling device when so loaded to the sampling device. It is envisaged that a tool may also be used to remove the cartridge.

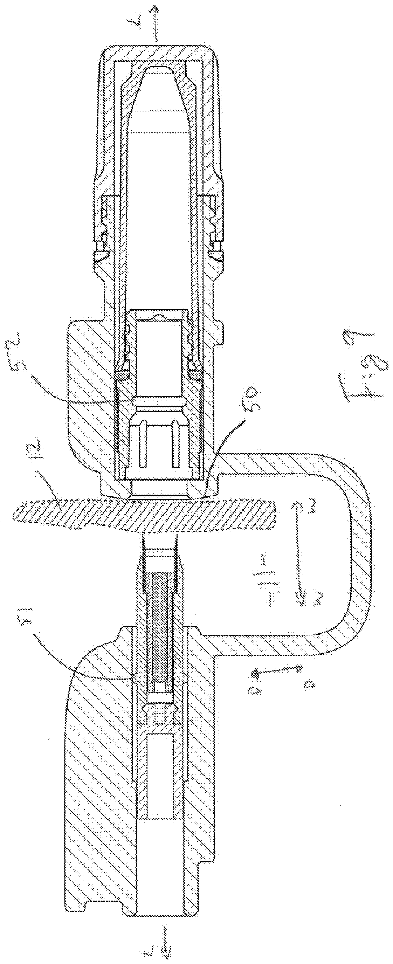

The sampling cartridge (2) preferably comprises of a sampling cartridge body (8) that in a pre-use condition holds a sample collector (9) and a storage container (10) in a spaced apart condition to define a gap (11) there between. At the gap (11), as can be seen in Figure 9, an item to be sampled (12) can be placed. The item to be sampled (12) may, for example, be part of an ear of an animal. The gap (11) is sufficiently wide in the direction WW to accommodate such an item as part of an ear of an animal. It is preferably also sufficiently deep in direction DD so that, for example, the ear of an animal can be located in the gap (11) and be aligned to the direction LL of the sampling components that will hereinafter be described, of the sampling cartridge (2). The gap is preferably U-shaped.

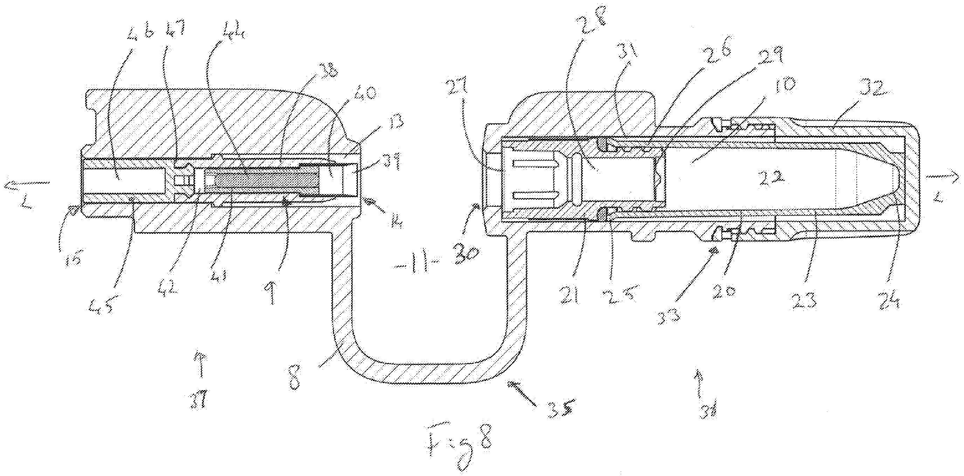

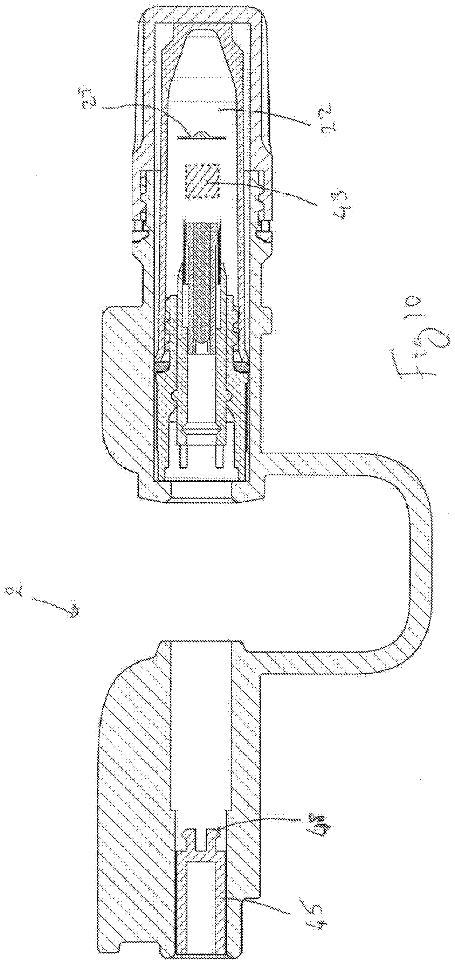

The sampling components of the sampling cartridge primarily include the sample collector (9) and the storage container (10). The sampling cartridge body (8) preferably holds these two components in alignment with each other in the pre sampling condition. In the preferred form, the sample collector is of an elongated form and as such the sampling cartridge body (8) preferably includes a bore in which the sample collector is located when in the pre sampling condition. Preferably the sample collector (9) is completely located within the bore (13) of the sample cartridge body (8). This can be seen in Figure 8. It is less desirable for part of the sample collector to be protruding from the bore because of the prospect then of pre sampling contamination of the sample collector (9). The bore at a gap end (14) may be sealed by way of a plug or a foil or the like to enclose the sample collector within the bore. At the opposed end (15) to the bore (13), a seal may also be provided. In the preferred form, a snug-fit relationship exists between the sample cartridge body (8) of the sampling cartridge at the bore (13) and the sample collector (9) so that the bore can guide the movement of the sampling cartridge as it moves from its pre-use condition as shown in Figure 8 to its sample storage container registered condition as shown in Figure 10. Preferably the shape and configuration of the sampling cartridge body and the storage container and sample collector is such that the sample collector (9) is guided by the bore (13) and/or by the storage container during its movement from its pre-use stored condition to its sample storage container registered condition.

At the other side of the gap (11) to where the sample collector (9) is located in the pre sampling and stored condition, is a storage container (10). The storage container (10) is removably engaged to the sampling cartridge body (8). The storage container (10) preferably includes a storage tube (20) and a removable cap (21) that is removably engaged to the storage tube (20). In the preferred form the removable cap (21) is threadingly engaged to the storage tube (20). However, in alternative forms the storage container (10) may be provided as a unitary body where no separate storage tube and cap are provided. The storage tube (20) preferably includes a containment region (22) at where ultimately a sample that has been collected by the sample collector is placed during the sampling process. The containment region (22) may include an additive such as a desiccant or the like (not shown). The containment region (22) is bounded by a tube side wall (23) and the end wall (24) at the mouth opening (25) of the storage tube (20) is where the cap (21) is located. As can be seen such location is by way of a threaded engagement (26). The storage container, preferably defined by the cap (21) when provided, includes an opening (27) via which the sample collector (9) can enter the storage container (10).

The opening (27) is sufficiently large to receive the sample collector (9). The opening (27) leads to a passage (28) of the cap (21). The passage (28) leads to the containment region (22). The passage may be occluded, in the pre sampling condition by a frangible web (29) that extends across the passage (28). The frangible web (29) is preferably of a construction that will allow for the sample collector (9) to sever it at least partially from the cap (21) and thereby open the containment region (22) and allow for at least part of the sample collector to enter into the containment region (22) or at least allow for the sample collected by the sample collector to be dispensed into the containment region (22). The frangible web (29), in the pre sampling condition helps ensure that no contaminants can enter into the containment region (22). In addition, or alternatively, a plug, or seal or foil may be placed at the opening (27) of the storage container. Alternatively, and in the configuration as shown where the storage container (10) is held by the sample cartridge body (8), the sample collected body may include an opening (30) that leads to the storage container (10). The opening (30) may be sealed in the pre sampling condition of the sampling cartridge (2) such as by way of a plug or foil seal, to prevent contaminants from reaching the opening (27) of the storage container (10). As can be seen in Figure 8 for example, the storage container (10) is preferably also of an elongate form in direction LL. In the preferred form, the sampling cartridge body (8) also includes a bore (31) in which at least part of the storage container (10) can be located and preferably snugly supported thereby. The bore (31) is preferably of a size so that it can rigidly and snugly hold, yet allow for removal, of the storage container (10) from the sampling cartridge body (8).

In the preferred form, the bore is capped by a cap (32) that can secure on to the sampling cartridge body (8). The cap (32) preferably encloses the storage container (10) within the sampling cartridge body (8). The cap (32) is removable from the sampling cartridge body (8) and preferably is threadingly engaged therewith. A tamper evident ring (33) is preferably provided so that an indication of the removal of the cap (32) from the sampling cartridge body can be indicated. The tamper evident ring (33) may be part of the cap (32) or part of the sampling cartridge body (8). Alternatively, it may be a foil seal that is located over the interface between the cap (32) and the sampling cartridge body (8).

Once the cap (32) has been removed from the sampling cartridge body, the storage container can be drawn out from the bore (31) to then remove the storage container from the sampling cartridge body. This will typically happen when sampling has occurred, the sample carrying sample collector (9) having been driven into the storage container (10) and retained thereby with the sample in the containment region (22). Such removal may occur, for example, at a laboratory. This means, for example, once a sample has been collected from an animal (eg from the ear of an animal) by a farmer, the entire sampling cartridge still retaining the storage container and sample holding sample collector may then be shipped to a laboratory as a single unit. At the laboratory, the cap (32) may be removed to gain access to the sample holding storage container and this may then be opened to gain access to the sample for analysis.

The sampling cartridge body (8) includes a trough region (35) that bridges the gap (11). The trough region (35) connects the storage container holding region (36) and the sample collector holding region (37) together. A bridge section extends across the trough region to connect the storage container holding region (36) and the sample collector holding region (37) together. The trough region (35) also provides a secondary purpose. During the process of sampling, hair and/or blood from the animal may shed from the animal and/or the sample collected from the animal. The trough region (35) provides shielding to the body (3) of the sampling device from such contaminants. This will help keep the body (3) of the sampling device clean or cleaner than it would otherwise be.

Preferred forms of the sample collector (9) and the storage container (10) will now be described.

In a preferred form, the sample collector (9) comprises of a punch (38) that at one end presents a cutter (39). The cutter may, for example, be of a cylindrical configuration and have a leading edge that is sharp for the purposes of penetrating into the tissue of an animal. The cutter (39) may include a bore (40) at where a sample can be retained upon the penetration of the cutter into the item from which the sample is to be removed. The bore (40) can retain the so removed sample with the punch (38). The punch (38) is preferably of an elongate configuration and is preferably of a cylindrical perimeter. In the preferred form, the punch may carry a plunger (41). The plunger (41) is preferably held in a bore (42) of the punch (38). The plunger can be caused to move within this bore.

In the preferred form, in the pre sampling condition the plunger is in a retracted condition as seen in Figure 8. In this retracted condition, the plunger and the cutter define the bore (40). The bore (40) is hence a blind bore. In an extended condition as seen in Figure 10, the plunger has moved relative to the punch and into the bore (40). This has caused the sample (43) to be ejected from the bore (40) and into the containment region (22). In the sample storage container registered condition as seen in Figure 10, the punch has also severed the frangible web (29) to open the containment region (22) to the sample (43). In the preferred form, the plunger (41) carries an ID such as an EID (44) that may, for example, be an RFID. This RFID carries a unique identifier that is machine readable and remains associated with the plunger (41), and hence the punch (38) of the sample collector (9).

In the preferred form, the plunger (41) and the punch (38) cannot be separated.

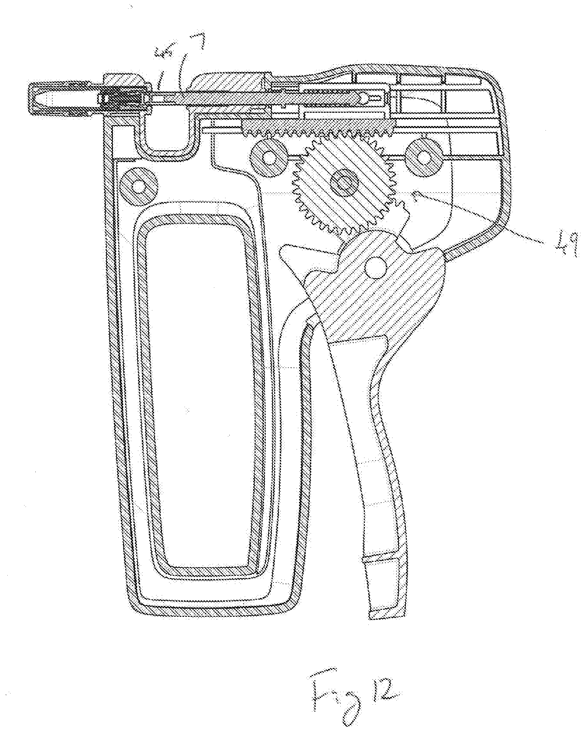

As can be seen in Figure 12, a cross-contamination sleeve/rod (45) may be provided in a removable association with the sample collector (9). The cross-contamination sleeve/rod provides an intermediary between the driver (7) of the sampling device (1) and the sample collector (9). The driver (7) may penetrate into the bore (46) of the cross-contamination sleeve/rod (45) in a manner to isolate it from contact with the item to be sampled. This hence provides a further measure against cross-contamination that might otherwise arise from contaminants touching the driver (7) of the sampling device (1). In the preferred form, the cross-contamination sleeve/rod (45) is engaged with the punch (38) at the driving end (47) of the punch so that it can drive the sample collector from its pre-use stored condition as seen in Figure 8 to its sample storage container registered condition as seen in Figure 10. The cross-contamination sleeve/rod may be removably engaged by way of a snapfit or interference fit configuration (48) that can registered at the driving end (47) of the punch (38). It can be released upon the retraction of the driver (7) and be carried back with the driver after the sample has been taken and the sample collector is in the storage container, as can for example be seen in Figure 10. Alternatively, the cross-contamination sleeve/rod (45) may be ejected from the sampling cartridge (2) after sampling.

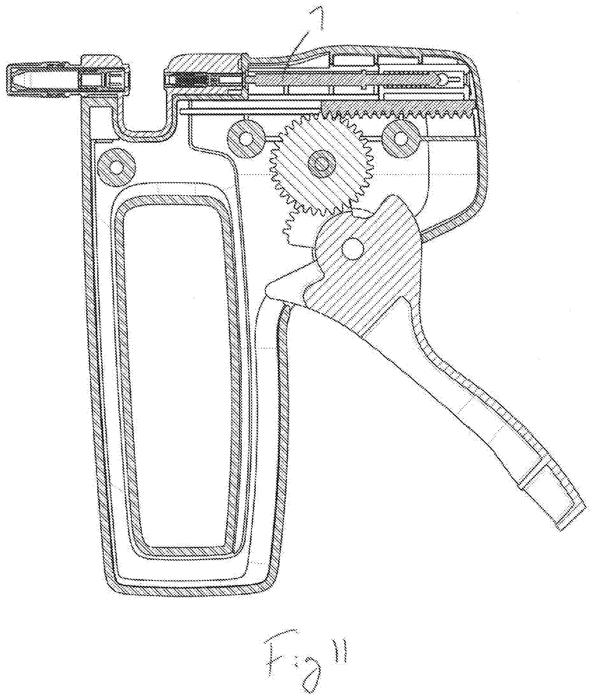

The driver (7) is preferably held relative to the body (3) in a manner to be actuable by the trigger (5) to cause it to move in a reciprocating manner between a retracted condition as seen in Figure 11, and an extended condition as seen in Figure 12. A rack and pinion-like arrangement (49) may be provided to facilitate the movement of the driver. A quick-release feature may be provided so that once the driver has been moved to its fully extended position a spring or other device may quickly retract the driver from its extended position back its retracted condition.

When a sample is being taken, the sample collector (9) may drive and/or press the item to be sampled (12) towards the storage container holding region of the sampling cartridge (2). As can be seen in Figure 9, the item to be sampled (12) is pressed against a die surface (50) of the storage container holding region (36) of the sampling cartridge body (8). The die surface (50) acts as a stop for the item to be sampled (12) as the sample collector is driven towards its sample storage container registered condition. The die surface (50) together with the cutter (39) act as a punch and die set to facilitate the removal of a sample from the item to be sampled (12). Hence in the preferred form, the opening (30) is sufficiently sized to allow for it to receive the cutter, and preferably the entire sample collector (9), yet act in a sheer fashion with the cutter (39) for the purposes of removing the sample. Hence the sizing of the opening (30) is sufficiently snug about the cutter to facilitate this sheering-like removal of the sample from the item to be sampled.

Upon a driving of the sample collector into the cap (21) of the storage container (10), the punch preferably locates very snugly within the passage (28) to thereby provide a seal to the passage (28). An interference fit ring (51) and groove (52) configuration may be provided by the punch (38) and cap (21) respectively, to facilitate such sealing. The ring and groove configuration will also help with retention of the sample collector with the storage container after the sample collector has been moved to its sample storage container registered condition, as can be seen in Figure 10.

To facilitate such snug/interference fit between the sample collector and the storage container, the material selection of one or both of the sample collector and the storage container (preferably the punch and the cap) needs to be such as to allow for such a snap-fit type relationship to be established. Therefore, preferably one or both of the punch and cap are of a relatively soft and preferably plastic material, to allow for a certain degree of deformation of features of one or both of these items to occur. Hence in the preferred form, the cap (21) is preferably of a plastics material and shape and configuration that can to some extent deform on its own, or preferably together with features of the sample collector to establish a snug, and preferably sealed relationship between the cap and the sample collector at the passage (28). In the preferred form, to ensure an efficient punch and die set-like relationship is established between the cutter (39) and the die surface (50), the die surface (50) is defined by a material which is rigid and preferably will not deform. In the preferred form, the die surface is part of the storage container body. Preferably, the die surface is of a material that is harder than that of the cap (21) of the storage container (10). Hence the present invention allows for an efficient punch and die set-like features to be provided for the purposes of taking the sample, without such being compromised by needing to use a material that may be softer of the cap of the storage container (10). Hence in the preferred form, the storage container cap is of a softer plastics material than the material used at the die surface (50) (preferably the material used for the storage container body). It will be appreciate that a separate insert may be provided to the storage container body to act as the die surface. A co-moulding of different plastics materials may be possible, or an insert of, for example, a metal insert may be provided for the purposes of providing the die surface (50).

One or both of the sampling cartridge body (8) and tube (20) may include a unique identifier. For example, a barcode or number or alphanumeric string may be provided on a surface of the sampling cartridge body. Alternatively, or additionally, an EID component may be provided on the sampling cartridge body. In addition, a unique barcode or other visual identifier or an EID may also be provided on the tube (20). This may for example be provided at the end wall (24) of the storage tube (20). Any one or more, and preferably all of the identifiers mentioned herein (EID/barcode or other) are preferably matched such a pre-matched to each other. It may be of the same identification or different identification that are matched with each other. The sample collector may also include such identifier. Preferably the identifiers relate to each other. They may be identical identifiers or via a database or other related to each other.

Upon the taking of a sample from an animal, a reading of an identifier of the animal (such as an RFID or visual ID of an ear tag of the animal) may be matched to the identifiers of the sampling cartridge (2) and/or tube and/or sample collector. This allows for a matching between the animal identification and the sample to be established.

In a laboratory, such matching can then verify whether the sample in the storage container is a valid sample that matches that of the animal from which the sample has been taken. The reading of the animal's identification may occur before, during or after the sample being taken from the animal.

In the preferred form, the cap (32) is of a transparent material. This allows for a visual inspection to occur to ensure that a sample is within the storage tube. Hence preferably the storage tube is also at least partially transparent.

## Claims

1. A sampling cartridge (2) comprising a sampling cartridge body (8) that includes a storage container holding region (36) at where a sample storage container (10) is able to be located and a sample collector holding region (13) at where a sample collector (9) is able to be located, wherein the sampling cartridge body (8) in use holds the sample collector (9) and the sample storage container (10) in alignment with each other in a pre-sampling condition spaced apart with a sufficient gap (11) to allow part of an item (12) to be sampled to locate at said gap (11), **characterised in that** the sample collector holding region (13) includes a bore (13) in which the sample collector (9) is located when in the pre-sampling condition.

2. The sampling cartridge (2) as claimed in claim 1 adapted and configured to be loadable and unloadable to and from a sampler device (1) that includes a driver (7) actuable to cause said in alignment sample collector (9) to move from sample collector holding region (13) across the gap (11) and into the sample storage container (10).

3. The sampling cartridge as claimed in claim 2 wherein a) the sampling cartridge body (8) is adapted and configured to shield the sampler device (1) at the gap (11) from contact with said item (12) to be sampled when placed at the gap (11), or b) the sampling cartridge body (8) is adapted and configured to shield the sampler device (1) at the gap (11) from contact with said item (12) to be sampled when placed at the gap (11), and any exudates expressed or expelled from said item during sampling.

4. The sampling cartridge (2) as claimed in any one of claims 1 to 3 comprising a bridge section (35) extending between said storage container holding region (36) and said sample collector holding region (13), said bridge section (35) spanning said gap (11), wherein said bridge section (35) provides said shield function.

5. The sampling cartridge (2) as claimed in any one of claims 1 to 4 wherein the gap (11) is substantially U shaped.

6. The sampling cartridge (2) as claimed in any one of claims 1 to 5 wherein said storage container holding region (36) and said sample collector holding region (13) are contiguous respective opposed sides of said gap (11).

7. The sampling cartridge (2) as claimed in claim any one of claims 1 to 6 wherein said storage container holding region (36) comprises a cavity (31) of said sampling cartridge body in which at least part of said sample storage container (10) is located.

8. The sampling cartridge (2) as claimed in claim 7 wherein a passage (28) leads from said cavity (31) to said gap (11) and via which the sample collector (9) can move to engage with said sample storage container (10).

9. The sampling cartridge (2) as claimed claim 8 wherein the passage (28) includes a mouth opening (25) presented at said gap (11) at where said sample collector (9) is configured to enter the passage (28), upon being provided with a sample taken from the item (12) to be sampled placed at the gap (11), to advance for engagement via an opening (27) of and into said sample storage container (10).

10. The sampling cartridge (2) as claimed in claim 9 wherein the material about said mouth opening (25) is harder than said material of the sample storage container (10) about its opening (27).

11. The sampling cartridge (2) as claimed in claim 9 or 10 wherein at said mouth opening (25) said sampling cartridge body (8) together with said sample collector (9) act as a punch and die set (38, 39; 50), the mouth opening (25) acting as the die (50) acting in shear with the sample collector (9) acting as said punch (38, 39) to facility the removal of a sample from the item (12) to be sampled.

12. The sampling cartridge (2) as claimed in any one of the preceding claims wherein at least one of the components selected from the following list:
a. Removable closure,
b. Storage container,
c. Sample collector,
d. Sampling cartridge body,
comprises at least one form of visible and/or machine readable identification.

13. A method of taking and storing a sample from an item (12) using the sampling cartridge (2) as claimed in any one of claims 12 comprising:
e. loading the sampling cartridge (2) to a sampler device (1) in the bore comprised in the sample collector holding region (13), so that the sample collector (9) and the sample storage container (10) are in alignment with a driver (7) of said sampler device (1), the driver to act, directly or indirectly on said sample collector (9),
f. placing a part of an item (12) to be sampled in said gap (11) so that part of the item (12) is also in alignment,
g. causing the driver (7) to move in a driving direction to drive said sample collector (9) from its holding region, across the gap (11) and towards said sample storage container (10) so that said sample collector (9) is driven into the sample storage container (10) so that said punch becomes held by said storage container,
h. causing the driver to move in a direction opposite said driving direction.

14. The method as claimed in claim 13 wherein a) identification carried by or of the item from which the sample is taken is recorded at the time of taking of the sample, or b) identification carried by or of the item from which the sample is taken is matched to the at least one form of visible and/or machine readable identification of said sampling cartridge.

## Patentansprüche

1. Probenkassette (2), umfassend einen Probenkassettenkörper (8), der einen Aufbewahrungsbehälteraufnahmebereich (36), an dem ein Probenaufbewahrungsbehälter (10) angeordnet werden kann, und einen Probensammleraufnahmebereich (13), an dem ein Probensammler (9) angeordnet werden kann, aufweist, wobei der Probenkassettenkörper (8) beim Gebrauch den Probensammler (9) und den Probenaufbewahrungsbehälter (10) zueinander ausgerichtet in einem Vorprobenentnahmezustand hält, der mit einem ausreichenden Zwischenraum (11) beabstandet ist, um zu ermöglichen, dass ein Teil eines zu beprobenden Gegenstands (12) in dem Zwischenraum (11) angeordnet ist, **dadurch gekennzeichnet, dass** der Probensammleraufnahmebereich (13) eine Bohrung (13) aufweist, in der der Probensammler (9) angeordnet ist, wenn dieser sich im Vorprobenentnahmezustand befindet.

2. Probenkassette (2) gemäß Anspruch 1, die so ausgelegt und gestaltet ist, dass sie in eine Probenentnahmevorrichtung (1) einbringbar und daraus ausbringbar ist, die eine Antriebsvorrichtung (7) aufweist, die betätigbar ist, um zu bewirken, dass der ausgerichtete Probensammler (9) aus dem Probensammleraufnahmebereich (13) über den Zwischenraum (11) und in den Probenaufbewahrungsbehälter (10) bewegt wird.

3. Probenkassette gemäß Anspruch 2, wobei a) der Probenkassettenkörper (8) so ausgelegt und gestaltet ist, dass dieser die Probenentnahmevorrichtung (1) in dem Zwischenraum (11) vor dem Kontakt mit dem zu beprobenden Gegenstand (12) abschirmt, wenn dieser in dem Zwischenraum (11) platziert ist, oder b) der Probenkassettenkörper (8) so ausgelegt und gestaltet ist, dass dieser die Probenentnahmevorrichtung (1) in dem Zwischenraum (11) vor dem Kontakt mit dem zu beprobenden Gegenstand (12) abschirmt, wenn dieser in dem Zwischenraum (11) platziert ist, und vor eventuellen Exsudaten abschirmt, die während der Probenentnahme aus dem Gegenstand ausgedrückt oder ausgestoßen werden.

4. Probenkassette (2) gemäß einem der Ansprüche 1 bis 3, umfassend einen Brückenabschnitt (35), der sich zwischen dem Aufbewahrungsbehälteraufnahmebereich (36) und dem Probensammleraufnahmebereich (13) erstreckt, wobei der Brückenabschnitt (35) den Zwischenraum (11) überspannt, wobei der Brückenabschnitt (35) die Abschirmfunktion bereitstellt.

5. Probenkassette (2) gemäß einem der Ansprüche 1 bis 4, wobei der Zwischenraum (11) im Wesentlichen U-förmig ist.

6. Probenkassette (2) gemäß einem der Ansprüche 1 bis 5, wobei der Aufbewahrungsbehälteraufnahmebereich (36) und der Probensammleraufnahmebereich (13) aneinandergrenzende bzw. gegenüberliegende Seiten des Zwischenraums (11) sind.

7. Probenkassette (2) gemäß einem der Ansprüche 1 bis 6, wobei der Aufbewahrungsbehälteraufnahmebereich (36) einen Hohlraum (31) des Probenkassettenkörpers umfasst, in dem sich mindestens ein Teil des Probenaufbewahrungsbehälters (10) befindet.

8. Probenkassette (2) gemäß Anspruch 7, wobei ein Durchgang (28) aus dem Hohlraum (31) zu dem Zwischenraum (11) führt und über den sich der Probensammler (9) bewegen kann, um mit dem Probenaufbewahrungsbehälter (10) in Eingriff zu kommen.

9. Probenkassette (2) gemäß Anspruch 8, wobei der Durchgang (28) eine sich in dem Zwischenraum (11) befindende Mündung (25) aufweist, an der der Probensammler (9) dazu ausgelegt ist, in den Durchgang (28) einzutreten, wenn er mit einer Probe versehen wird, die aus dem zu beprobenden Gegenstand (12) entnommen wurde, der in dem Zwischenraum (11) platziert ist, um sich zum Eingriff über eine Öffnung (27) des Probenaufbewahrungsbehälters (10) und in diesen hinein vorwärts zu bewegen.

10. Probenkassette (2) gemäß Anspruch 9, wobei das Material um die Mündung (25) härter als das Material des Probenaufbewahrungsbehälters (10) um dessen Öffnung (27) ist.

11. Probenkassette (2) gemäß Anspruch 9 oder 10, wobei an der Mündung (25) der Probenkassettenkörper (8) zusammen mit dem Probensammler (9) als Stempel- und Matrizensatz (38, 39; 50) wirkt, wobei die Mündung (25) als Matrize (50) wirkt, die unter Scherung mit dem Probensammler (9) wirkt, der als Stempel (38, 39) wirkt, um die Entnahme einer Probe aus dem zu beprobenden Gegenstand (12) zu ermöglichen.

12. Probenkassette (2) gemäß einem der vorhergehenden Ansprüche, wobei mindestens eine der Komponenten, die aus der folgenden Liste ausgewählt ist:
a. Abnehmbarer Verschluss,
b. Aufbewahrungsbehälter,
c. Probensammler,
d. Probenkassettenkörper,
mindestens eine Form von sichtbarer und/oder maschinenlesbarer Kennzeichnung aufweist.

13. Verfahren zum Entnehmen und Aufbewahren einer Probe aus einem Gegenstand (12) unter Verwendung der Probenkassette (2) gemäß einem der Ansprüche 12, umfassend:
e. Einbringen der Probenkassette (2) in eine Probensammlervorrichtung (1) in der Bohrung, die in dem Probensammleraufnahmebereich (13) enthalten ist, sodass der Probensammler (9) und der Probenaufbewahrungsbehälter (10) mit einer Antriebsvorrichtung (7) der Probenentnahmevorrichtung (1) ausgerichtet sind, wobei die Antriebsvorrichtung direkt oder indirekt auf den Probensammler (9) einwirkt,
f. Platzieren eines Teils eines zu beprobenden Gegenstands (12) in dem Zwischenraum (11), sodass ein Teil des Gegenstands (12) ebenfalls ausgerichtet ist,
g. Bewirken, dass sich die Antriebsvorrichtung (7) in eine Antriebsrichtung bewegt, um den Probensammler (9) aus seinem Aufnahmebereich über den Zwischenraum (11) und in Richtung des Probenaufbewahrungsbehälters (10) zu treiben, sodass der Probensammler (9) in den Probenaufbewahrungsbehälter (10) getrieben wird, sodass der Stempel durch den Aufbewahrungsbehälter gehalten wird,
h. Bewirken, dass sich die Antriebsvorrichtung in eine zur Antriebsrichtung entgegengesetzte Richtung bewegt.

14. Verfahren gemäß Anspruch 13, wobei a) die durch den Gegenstand, aus dem die Probe entnommen wird, getragene Kennzeichnung zum Zeitpunkt der Entnahme der Probe aufgezeichnet wird, oder b) die durch den Gegenstand, aus dem die Probe entnommen wird, getragene Kennzeichnung mit der mindestens einen Form sichtbarer und/oder maschinenlesbarer Kennzeichnung der Probenkassette abgeglichen wird.

## Revendications

1. Cartouche d'échantillonnage (2) comprenant un corps de cartouche d'échantillonnage (8) qui comporte une région de maintien de récipient de stockage (36) à l'endroit où un récipient de stockage d'échantillon (10) peut être situé et une région de maintien de collecteur d'échantillon (13) à l'endroit où un collecteur d'échantillon (9) peut être situé, où le corps de cartouche d'échantillonnage (8), en utilisation, maintient le collecteur d'échantillon (9) et le récipient de stockage d'échantillon (10) alignés l'un avec l'autre dans un état de pré-échantillonnage séparé par un espace suffisant (11) pour permettre à une partie d'un article (12) à échantillonner d'être placée au niveau dudit espace (11), **caractérisée en ce que** la région de maintien de collecteur d'échantillon (13) comporte un alésage (13) dans lequel le collecteur d'échantillon (9) est positionné lorsqu'il se trouve dans l'état de pré-échantillonnage.

2. Cartouche d'échantillonnage (2) telle que revendiquée dans la revendication 1, adaptée et configurée pour pouvoir être chargée et déchargée vers et depuis un dispositif échantillonneur (1) qui comporte une unité d'entraînement (7) actionnable pour amener ledit collecteur d'échantillon en alignement (9) à se déplacer de la région de maintien de collecteur d'échantillon (13) à travers l'espace (11) et dans le récipient de stockage d'échantillon (10).

3. Cartouche d'échantillonnage telle que revendiquée dans la revendication 2, dans laquelle a) le corps de cartouche d'échantillonnage (8) est adapté et configuré pour protéger le dispositif échantillonneur (1) au niveau de l'espace (11) d'un contact avec ledit article (12) à échantillonner lorsqu'il est placé au niveau de l'espace (11), ou b) le corps de cartouche d'échantillonnage (8) est adapté et configuré pour protéger le dispositif échantillonneur (1) au niveau de l'espace (11) d'un contact avec ledit article (12) à échantillonner lorsqu'il est placé au niveau de l'espace (11), et tout exsudat exprimé ou expulsé dudit article lors de l'échantillonnage.

4. Cartouche d'échantillonnage (2) telle que revendiquée dans l'une quelconque des revendications 1 à 3, comprenant une section de pont (35) s'étendant entre ladite région de maintien de récipient de stockage (36) et ladite région de maintien de collecteur d'échantillon (13), ladite section de pont (35) occupant ledit espace (11), où ladite section de pont (35) assure ladite fonction de protection.

5. Cartouche d'échantillonnage (2) telle que revendiquée dans l'une quelconque des revendications 1 à 4, dans laquelle l'espace (11) est sensiblement en forme de U.

6. Cartouche d'échantillonnage (2) telle que revendiquée dans l'une quelconque des revendications 1 à 5, dans laquelle ladite région de maintien de récipient de stockage (36) et ladite région de maintien de collecteur d'échantillon (13) sont des côtés opposés respectifs contigus dudit espace (11).

7. Cartouche d'échantillonnage (2) telle que revendiquée dans l'une quelconque des revendications 1 à 6, dans laquelle ladite région de maintien de récipient de stockage (36) comprend une cavité (31) dudit corps de cartouche d'échantillonnage dans laquelle se trouve au moins une partie dudit récipient de stockage d'échantillon (10).

8. Cartouche d'échantillonnage (2) telle que revendiquée dans la revendication 7, dans laquelle un passage (28) mène de ladite cavité (31) audit espace (11) et par lequel le collecteur d'échantillon (9) peut se déplacer pour s'engager avec ledit récipient de stockage d'échantillon (10).

9. Cartouche d'échantillonnage (2) telle que revendiquée dans la revendication 8, dans laquelle le passage (28) comporte une ouverture d'embouchure (25) présentée au niveau dudit espace (11) à l'endroit où ledit collecteur d'échantillon (9) est configuré pour entrer dans le passage (28), lorsqu'il reçoit un échantillon prélevé sur l'article (12) à échantillonner placé au niveau de l'espace (11), pour avancer pour l'engagement via une ouverture (27) dudit récipient de stockage d'échantillon (10) et dans celui-ci.

10. Cartouche d'échantillonnage (2) telle que revendiquée dans la revendication 9, dans laquelle le matériau autour de ladite ouverture d'embouchure (25) est plus dur que ledit matériau du récipient de stockage d'échantillon (10) autour de son ouverture (27).

11. Cartouche d'échantillonnage (2) telle que revendiquée dans la revendication 9 ou 10, dans laquelle, au niveau de ladite ouverture d'embouchure (25), ledit corps de cartouche d'échantillonnage (8) conjointement avec ledit collecteur d'échantillon (9) agissent comme un ensemble poinçon et matrice (38, 39 ; 50), l'ouverture d'embouchure (25) agissant comme la matrice (50) agissant en cisaillement avec le collecteur d'échantillon (9) agissant comme ledit poinçon (38, 39) pour faciliter le retrait d'un échantillon de l'article (12) à échantillonner.

12. Cartouche d'échantillonnage (2) telle que revendiquée dans l'une quelconque des revendications précédentes, dans laquelle au moins l'un des composants sélectionné dans la liste suivante :
a. une fermeture amovible,
b. un récipient de stockage,
c. un collecteur d'échantillon,
d. un corps de cartouche de prélèvement,
comprend au moins une forme d'identification visible et/ou lisible par machine.

13. Procédé de prélèvement et de stockage d'un échantillon provenant d'un article (12) en utilisant la cartouche d'échantillonnage (2) telle que revendiquée dans l'une quelconque des revendications précédentes comprenant le fait de :
e. charger la cartouche d'échantillonnage (2) dans un dispositif échantillonneur (1) dans l'alésage compris dans la région de maintien de collecteur d'échantillon (13), de sorte que le collecteur d'échantillon (9) et le récipient de stockage d'échantillon (10) soient alignés avec une unité d'entraînement (7) dudit dispositif échantillonneur (1), l'unité d'entraînement devant agir, directement ou indirectement, sur ledit collecteur d'échantillon (9),
f. placer une partie d'un article (12) à échantillonner dans ledit espace (11) de sorte que cette partie de l'article (12) soit également alignée,
g. amener l'unité d'entraînement (7) à se déplacer dans une direction d'entraînement pour entraîner ledit collecteur d'échantillon (9) depuis sa région de maintien, à travers l'espace (11) et vers ledit récipient de stockage d'échantillon (10) de sorte que ledit collecteur d'échantillon (9) soit entraîné dans le récipient de stockage d'échantillon (10) de sorte que ledit poinçon soit maintenu par ledit récipient de stockage,
h. amener l'unité d'entraînement à se déplacer dans une direction opposée à ladite direction d'entraînement.

14. Procédé tel que revendiqué dans la revendication 13, dans lequel a) l'identification portée par ou de l'article dont l'échantillon est prélevé est enregistrée au moment du prélèvement de l'échantillon, ou b) l'identification portée par ou de l'article dont l'échantillon est prélevé est adaptée à l'au moins une forme d'identification visible et/ou lisible par machine de ladite cartouche d'échantillonnage.
